(19) 

**Europäisches Patentamt**
**European Patent Office**
**Office européen des brevets**

(11) **EP 3 917 386 B1**

(12) **EUROPÄISCHE PATENTSCHRIFT**

(45) Veröffentlichungstag und Bekanntmachung des Hinweises auf die Patenterteilung:
**05.03.2025 Patentblatt 2025/10**

(21) Anmeldenummer: **20702989.3**

(22) Anmeldetag: **29.01.2020**

(51) Internationale Patentklassifikation (IPC):
**A61B 5/021** *(2006.01)* **A61B 5/026** *(2006.01)*
**A61B 5/00** *(2006.01)*

(52) Gemeinsame Patentklassifikation (CPC):
**A61B 5/02116; A61B 5/0261; A61B 5/7239;**
**A61M 1/14;** A61B 5/6816; A61B 5/6824;
A61B 5/6826; A61B 5/6829; A61B 5/6831;
A61B 5/7264; A61M 2205/3561; A61M 2205/3592;
A61M 2230/04; A61M 2230/30

(86) Internationale Anmeldenummer:
**PCT/EP2020/052138**

(87) Internationale Veröffentlichungsnummer:
**WO 2020/157114 (06.08.2020 Gazette 2020/32)**

(54) **VERFAHREN ZUM BESTIMMEN EINES BLUTDRUCKWERTS EINES PATIENTEN, BLUTDRUCKMESSGERÄT UND DIALYSESYSTEM**

METHOD FOR DETERMINING A BLOOD PRESSURE VALUE OF A PATIENT, BLOOD PRESSURE METER AND DIALYSIS SYSTEM

PROCÉDÉ DE DÉTERMINATION D'UNE VALEUR DE PRESSION ARTÉRIELLE D'UN PATIENT, TENSIOMÈTRE ET SYSTÈME DE DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.01.2019 DE 102019102178**

(43) Veröffentlichungstag der Anmeldung:
**08.12.2021 Patentblatt 2021/49**

(73) Patentinhaber: **Fresenius Medical Care Deutschland GmbH**
**61352 Bad Homburg (DE)**

(72) Erfinder:
• **WEI, Zhang**
**97464 Niederwerrn (DE)**
• **RÜTH, Christof**
**97762 Hammelburg (DE)**

(74) Vertreter: **Nordmeyer, Philipp Werner**
**Maucher Jenkins**
**Patent- und Rechtsanwälte**
**Liebigstraße 39**
**80538 München (DE)**

(56) Entgegenhaltungen:
**EP-A1- 3 427 651 WO-A1-2015/092753**
**GB-A- 2 552 455**

• **GU W B ET AL: "A novel parameter from PPG dicrotic notch for estimation of systolic blood pressure using pulse transit time", MEDICAL DEVICES AND BIOSENSORS, 2008. ISSS-MDBS 2008. 5TH INTERNATIONAL SUMMER SCHOOL AND SYMPOSIUM ON, IEEE, PISCATAWAY, NJ, USA, 1 June 2008 (2008-06-01), pages 86 - 88, XP031341003, ISBN: 978-1-4244-2252-4**

- KACHUEE MOHAMMAD ET AL: "Cuffless Blood Pressure Estimation Algorithms for Continuous Health-Care Monitoring", IEEE TRANSACTIONS ON BIOMEDICAL ENGINEERING, IEEE SERVICE CENTER, PISCATAWAY, NJ, USA, vol. 64, no. 4, 1 April 2017 (2017-04-01), pages 859 - 869, XP011642867, ISSN: 0018-9294, [retrieved on 20170317], DOI: 10.1109/TBME.2016.2580904
- QAWQZEH YOUSEF K ET AL: "Photoplethysmogram reflection index and aging", INTERNATIONAL CONFERENCE ON GRAPHIC AND IMAGE PROCESSING (ICGIP 2011), SPIE, 1000 20TH ST. BELLINGHAM WA 98225-6705 USA, vol. 8285, no. 1, 1 October 2011 (2011-10-01), pages 1 - 6, XP060019320, DOI: 10.1117/12.913587

- Q. YOUSEF ET AL: "The Analysis of PPG Morphology: Investigating the Effects of Aging on Arterial Compliance", MEASUREMENT SCIENCE REVIEW, vol. 12, no. 6, 31 December 2012 (2012-12-31), XP055294424, DOI: 10.2478/ v10048-012-0036-3

**Beschreibung**

Technisches Gebiet

Die vorliegende Erfindung betrifft ein Verfahren zum Bestimmen eines Blutdruckwerts eines Patienten und ein entsprechendes Blutdruckmessgerät. Weiterhin betrifft die Erfindung ein Dialysesystem, umfassend ein derartiges Blutdruckmessgerät.

Stand der Technik

Aus dem Stand der Technik sind Dialysevorrichtungen zur Durchführung einer Hämodialyse bekannt, bei denen Blut eines Patienten in einem extrakorporalen Kreislauf durch eine Dialysatoreinheit geführt und so von unerwünschten Substanzen gereinigt wird und eine Reduktion des Körperwassers durchgeführt wird.

Bei der Hämodialyse findet in der Dialysatoreinheit ein Austausch von Substanzen und Flüssigkeiten entlang einer semipermeablen Membran, auch als Dialysator bezeichnet, durch Diffusion mithilfe jeweiliger Konzentrationsgradienten zwischen dem Blut des Patienten und einem entsprechend ausgestalteten Dialysat statt.

Weiterhin sind Dialysevorrichtungen zur Durchführung einer Hämofiltration bekannt, bei welchen in einem extrakorporalen Kreislauf dem Blut Substanzen und Flüssigkeitsvolumen durch eine mit einem Druckgradienten beaufschlagte semipermeable Membran des Dialysators entzogen werden. Um den durch die Porengröße der Membran bedingten erhöhten Entzug von Flüssigkeitsvolumen, Salzen und anderen Substanzen aus dem Blut nach dem Durchließen des Dialysators wieder auszugleichen, wird dem Blut typischer Weise nach dem Durchfließen des Dialysators eine Substitutionsflüssigkeit zugeführt.

Es ist auch eine Kombination der beiden Verfahren bekannt, die dann als Hämodiafiltration bezeichnet wird.

Bei der Dialyse wird dem Patienten entsprechend Wasser, insbesondere Plasmawasser, entzogen, wodurch das Herz-Kreislaufsystem des Patienten, insbesondere durch Reduktion des Blutvolumens, beeinflusst werden kann.

Intradialytische Hypotension gilt als eine der häufigsten Komplikationen bei der Durchführung der Hämodialyse. Diese kann zu Übelkeit, Erbrechen Kopfschmerzen, Schwindel, Krämpfen, Müdigkeit, Hautblässe, Kaltschweißigkeit und/oder zur Bewusstlosigkeit des Patienten führen und bedarf entsprechend einer Pflegeintervention. Eine Ursache hierfür kann ein zu hoher oder zu schneller Wasserentzug aus dem Blut des Patienten und/oder ein mangelnder Zustrom von Substitutionsflüssigkeit in das Blut, sogenanntes "Refilling", sein.

Als Indikator für die intradialytische Hypotension kann eine Abnahme des systolischen Blutdrucks und/oder diastolischen Blutdrucks und/oder eine Abnahme des mittleren arteriellen Blutdrucks herangezogen werden.

Bei bekannten Dialysegeräten werden Blutdruckwerte, insbesondere ein systolischer Blutdruckwert, in der Regel intermittierend, insbesondere stündlich, mit einem Manschetten-basierten, nicht-invasiven Blutdruckmonitor oszillometrisch gemessen. Da es sich hierbei jedoch um ein nichtkontinuierliches Verfahren handelt und ein akuter Blutdruckabfall schnell, beispielsweise innerhalb von 10 Minuten, erfolgen kann, ist ein derartiges Messverfahren für eine frühzeitige und zuverlässige Erkennung einer Intradialytischen Hypotension wenig geeignet.

Aus KACHUEE MOHAMMAD ET AL: "Cuffless Blood Pressure Estimation Algorithms for Continuous Health-Care Monitoring", IEEE TRANSACTIONS ON BIOMEDICAL ENGINEERING, IEEE SERVICE CENTER, PISCATAWAY, NJ, USA, Bd. 64, Nr. 4, 1. April 2017 (2017-04-01), Seiten 859-869, XP011642867, ISSN: 0018-9294, DOI: 10.1109/TBME.2016.2580904, ist ein Verfahren zum manschettenlosen Überwachen eines ermittelten Blutdrucks bekannt, wobei physiologische Parameter aus Signalen eines Photoplethysmographs extrahiert werden.

Eine direkte Überwachung des Blutdrucks mittels einer Manschette ist aus der EP 3 427 651 A1 bekannt. Weiterhin offenbart die GB 2 552 455 A eine Messung des Blutdrucks mittels einer reflektierten Druckwelle anhand einer dikrotischen Kerbe. Aus der WO 2015/092753 A1 geht weiterhin ein Bestimmen des Blutdrucks anhand von Pulswellenparametern hervor.

Darstellung der Erfindung

Ausgehend von dem bekannten Stand der Technik ist es eine Aufgabe der vorliegenden Erfindung, ein verbessertes Verfahren zum Bestimmen eines Blutdruckwerts eines Patienten, sowie ein entsprechendes Blutdruckmessgerät für ein Dialysesystem bereitzustellen, die insbesondere eine kontinuierliche und anhaltende Überwachung des Blutdruckwerts des Patienten ermöglichen und kostengünstig implementierbar sind.

Die Aufgabe wird durch ein Verfahren zum Bestimmen eines Blutdruckwerts eines Patienten mit den Merkmalen des Anspruchs 1, ein Blutdruckmessgerät mit den Merkmalen des Anspruchs 8 und ein Dialysesystem mit den Merkmalen des Anspruchs 11 gelöst. Vorteilhafte Weiterbildungen ergeben sich aus den Unteransprüchen, der Beschreibung und den Figuren.

Entsprechend wird ein Verfahren zum Bestimmen eines Blutdruckwerts eines Patienten vorgeschlagen, umfassend einen Schritt des Ermittelns eines Pulsverlaufs, insbesondere eines zeitabhängigen Pulsverlaufs, des Patienten und einen Schritt des Bestimmens eines Referenzpunktes im Pulsverlauf in einem Übergangsbereich von der Systole zur Diastole eines Herzzyklus des Patienten. In dem Verfahren wird weiterhin der Blutdruckwert auf der Grundlage des bestimmten Referenzpunktes ermittelt.

Indem bei dem vorgeschlagenen Verfahren ein Ermitteln des Blutdruckwerts auf der Grundlage eines zu ermittelnden Pulsverlaufs erfolgt, kann eine Überwachung eines Blutdruckwerts durchgehend über einen längeren Zeitraum erfolgen, beispielsweise während einer gesamten Behandlungszeit bei einem Dialyseverfahren. Weiterhin kann das hier vorgeschlagene Verfahren aufwandsreduziert durchgeführt werden im Vergleich zu den in bekannten Dialysevorrichtungen zum Einsatz kommenden oszillometrischen Messverfahren, die mittels eines Manschetten-basierten Blutdruckmonitors erfolgen.

Das Ermitteln des Blutdruckwerts des Patienten auf der Grundlage des Pulsverfahrens wird dadurch ermöglicht, dass in dem vorgeschlagenen Verfahren das Bestimmen eines Referenzpunktes in dem Pulsverlauf vorgeschlagen wird, der eine eindeutige und zuverlässige Korrelation zu einem dazugehörigen Blutdruckwert zulässt. Mit anderen Worten lässt sich so in Abhängigkeit des Referenzpunktes ein eindeutiger Blutdruckwert zuverlässig bestimmen.

Genauer wird hierbei die Bestimmung eines Referenzpunktes vorgeschlagen, der einen Übergangsbereich von der Systole zur Diastole eines Herzzyklus des Patienten anzeigt.

Im Allgemeinen werden unter den Begriffen "Systole" und "Diastole" unterschiedliche Phasen des Herzzyklus verstanden. Genauer handelt es sich bei der Systole um diejenige Phase, in der der Herzmuskel angespannt und somit Blut aus den Herzkammern in das Blutkreislaufsystem gepumpt wird. Demgegenüber beschreibt die Diastole die Entspannungsphase des Herzens, während der Blut in die Herzkammern strömt. Der Phasenübergang des Herzzyklus von der Systole zu der Diastole wird auch als Inzisur bezeichnet, der dem Zeitpunkt des Schließens der Aortenklappe, auch Taschenklappe genannt, entspricht.

In einer Pulskurve kann der Zeitbereich, in dem die Aortenklappe schließt, darüber erkannt werden, dass es aufgrund hydrodynamischer Effekte wie beispielsweise der Elastizität der Gefäßwände oder einem Windkesseleffekt dabei zu einem kurzfristigen Ansteigen der Pulskurve kommt. Auf diese Weise lässt sich die Inzisur bei einem gesunden Herzen in einem Bereich zwischen der Systole und der Diastole erkennen.

Wie vorangehend beschrieben, liegt der zu bestimmende Referenzpunkt in dem Übergangsbereich von der Systole zu Diastole eines Herzzyklus des Patienten. Unter dem Übergangsbereich von der Systole zu Diastole wird hierbei ein Bereich in dem Pulsverlauf verstanden, in dem der Phasenübergang von der Systole zu der Diastole des Herzzyklus des Patienten erfolgt. Mit anderen Worten liegt in diesem Bereich die Inzisur des Herzzyklus.

Durch den Referenzpunkt wird, wie dessen Bezeichnung zu entnehmen ist, eine eindeutige Bezugsgröße für den Pulsverlauf eines Herzzyklus bereitgestellt, die insbesondere eine Vergleichbarkeit zwischen Pulsverläufen unterschiedliche Herzzyklen eines Patienten ermöglicht. Entsprechend kann der Referenzpunkt ein mathematisch eindeutig definierbarer Punkt in dem Pulsverlauf sein, insbesondere in dem Übergangsbereich des Pulsverlaufs.

Indem der Referenzpunkt in dem Übergangsbereich von der Systole zu der Diastole des Herzzyklus liegt, kann gewährleistet werden, dass sich der Referenzpunkt zumindest näherungsweise an der Inzisur orientiert und mit dieser in Verbindung steht oder mit dieser zeitlich gekoppelt ist. Mit anderen Worten kann der Referenzpunkt zeitlich im Bereich der Inzisur des Herzzyklus liegen oder möglichst mit der Inzisur zeitlich übereinstimmen.

In dem vorgeschlagenen Verfahren ist der Übergangsbereich von der Systole zu Diastole des Herzzyklus des Patienten zwischen einem lokalen Minimum und einem lokalen Maximum in dem Pulsverlauf angeordnet. Insbesondere erstreckt sich der Übergangsbereich zwischen dem lokalen Minimum und dem lokalen Maximum.

Zum Festlegen des Übergangsbereichs kann das Verfahren ferner einen Schritt des Bestimmens des lokalen Minimums und des lokalen Maximums umfassen. Dieser Schritt kann in Abhängigkeit einer Änderungsrate des ermittelten Pulsverlaufs erfolgen. Die Änderungsrate des Pulsverlaufs entspricht dabei einer ersten Ableitung des Pulsverlaufs, wobei das lokale Minimum und das lokale Maximum des Pulsverlaufs eine Änderungsrate von null aufweisen. Mit anderen Worten können über eine Nullwertbestimmung in der Änderungsrate das lokale Minimum und das lokale Maximum in dem Pulsverlauf ermittelt werden.

Das hier vorgeschlagene Verfahren kann zum Bestimmen und Überwachen eines Blutdruckwerts eines Patienten während einer Dialysebehandlung, insbesondere während einer Hämodialyse, verwendet werden. Entsprechend kann das Verfahren in Verbindung mit einem Dialysegerät zur Anwendung kommen. Das vorgeschlagene Verfahren ist jedoch nicht auf diese Anwendung beschränkt. Vielmehr kann das Verfahren in einer beliebigen Anwendung zum Einsatz kommen, in der eine Bestimmung eines Blutdruckwerts des Patienten erfolgt oder erforderlich ist.

Genauer kann das Verfahren dazu vorgesehen sein, als Blutdruckwert einen systolischen Blutdruck zu bestimmen. Im Allgemeinen wird unter dem Begriff "systolischer Blutdruck" ein maximaler Wert des Blutdrucks während eines Herzzyklus verstanden.

Alternativ oder zusätzlich kann das Verfahren dazu vorgesehen sein, als Blutdruckwert einen mittleren arteriellen Druck zu bestimmen. Unter dem Begriff "mittlerer arterieller Druck" wird im Allgemeinen ein Mittelwert einer Blutdruckkurve über die Zeit, insbesondere über einen Herzzyklus, verstanden.

Alternativ oder zusätzlich kann das Verfahren dazu vorgesehen sein, einen diastolischen Blutdruck zu bestimmen. Im Allgemeinen wird unter dem Begriff "diastolischer Blutdruck" ein minimaler Wert des Blutdrucks während eines Herzzyklus verstanden.

Der zu bestimmende Blutdruckwert kann ein absoluter Wert für den Blutdruck sein. Alternativ kann der zu bestimmende Blutdruckwert ein relativer Blutdruckwert sein, insbesondere bezogen auf einen Referenzblutdruckwert. Indem der zu bestimmende Blutdruckwert in Form eines relativen Blutdruckwerts bereitgestellt wird, kann eine Änderung des Blutdruckwerts mittels des Verfahrens angezeigt werden. Der Blutdruckreferenzwert kann beispielsweise einem Wert des Blutdrucks in einem vorangehenden Herzzyklus entsprechen. Beispielsweise kann der Blutdruckreferenzwert einem Blutdruckwert zu Beginn einer Überwachung oder zu Beginn eines medizinischen Behandlungsverfahrens, insbesondere eines Dialyseverfahrens entsprechen.

Wie vorangehend beschrieben umfasst das Verfahren den Schritt des Ermittelns des zeitabhängigen Pulsverlaufs des Patienten. Im Allgemeinen werden unter dem Begriff "Puls" die mechanischen Auswirkungen der durch den systolischen Blutausstoß stammenden Druck- und Volumenschwankungen im Blutkreislaufsystem des Körpers verstanden. Die unterschiedlichen mechanischen Auswirkungen zeigen sich hierbei sowohl in der Frequenz der Druckstöße, in deren Amplitude und deren Verlauf. Unter einem "Pulsverlauf" kann vorliegend ein zeitlicher Verlauf der Amplitude des Pulses an einer Körperstelle, beispielsweise an einem Arm des Patienten verstanden werden.

Der Pulsverlauf kann derart ermittelt werden, dass dieser einen Herzzyklus des Patienten zumindest teilweise anzeigt. Alternativ kann der Pulsverlauf derart ermittelt werden, dass dieser wenigstens einen Herzzyklus des Patienten, vorzugsweise mehrere, insbesondere aufeinanderfolgende Herzzyklen des Patienten anzeigt. Das Ermitteln des Pulsverlaufs kann dabei kontinuierlich, insbesondere ohne Unterbrechungen erfolgen. Der im Verfahren ermittelte Pulsverlauf kann entsprechend einen kontinuierlichen und/oder stetigen Verlauf des Pulses, insbesondere der Pulsamplitude, abbilden. Alternativ oder zusätzlich kann das Ermitteln des Pulsverlaufs intermittierend, insbesondere in voneinander unterbrochenen Zeitabschnitten, erfolgen.

In einer Weiterentwicklung kann der Pulsverlauf auf der Grundlage von zeit- und volumenabhängigen Durchblutungswerten an einem Gewebeabschnitt des Patienten erfasst werden. Dies kann mittels Photoplethysmographie erfolgen. Unter dem Begriff "Photoplethysmographie" wird im Allgemeinen ein optisches Messverfahren zum Bestimmen einer Volumenänderung eines Organs, eines durchbluteten Gewebeabschnitts oder eines Blutgefäßes verstanden, die abhängig ist von der im Blutkreislauf auftretenden Blutdruckwelle. Hierbei wird in der Regel mittels eines Emitters Licht im roten oder Infrarotwellenbereich in lebendes Gewebe hineingestrahlt und der Grad der Lichtabsorption mittels eines Detektors gemessen, der anhand der veränderten Lichtabsorption einen volumenabhängigen Messwert liefert. Mit anderen Worten nimmt der Absorptionsgrad des Gewebes mit der Systole zu und mit der Diastole ab, so dass die gemessene Lichtabsorption eine Messung des Blutvolumenpulses ermöglicht.

Genauer kann das Messen der Lichtabsorption mittels eines photoplethysmografischen Sensors, z.B. mittels eines Pulsoximeters, erfolgen. Hierbei kann zwischen Sensoren unterschieden werden, die die Lichtabsorption mittels durch das Gewebe transmittierten Lichts oder mittels an dem Gewebe reflektierten Lichts bestimmen. Dies kann insbesondere an einem gut durchbluteten Gewebeabschnitt des Patienten erfolgen, beispielsweise an einem Arm, insbesondere Unterarm, einem Finger, einem Zeh oder einem Ohrläppchen des Patienten.

In einer Weiterentwicklung kann der Pulsverlauf normiert werden. Hierzu kann das Verfahren einen weiteren Schritt des Normierens des ermittelten oder gemessenen Pulsverlaufs umfassen. Dies ermöglicht die Genauigkeit der Bestimmung des Blutdruckwerts zu verbessern.

Beispielsweise kann der gemessene Pulsverlauf auf der Grundlage einer Amplitudenhöhe, insbesondere einer innerhalb eines Herzzyklus maximalen Amplitudenhöhe, normiert werden. Entsprechend kann der Pulsverlauf ein amplitudennormierter Pulsverlauf sein. Auf diese Weise kann die Anfälligkeit der Amplitudenschwankungen des gemessenen Pulsverlaufs minimiert werden.

Alternativ oder zusätzlich kann der Pulsverlauf zeitnormiert sein. Beispielsweise kann der Pulsverlauf durch eine jeweilige Herzzyklusdauer oder -periode normiert sein. Die Zeitnormierung durch die Herzzyklusdauer kann den Effekt aufweisen, dass eine Auswirkung der Herzfrequenzvariation auf die Bestimmung des Referenzpunktes eliminiert werden kann.

Wie vorangehend beschrieben kann der Referenzpunkt ein mathematisch bestimmbarer, insbesondere eindeutig bestimmbarer Punkt in dem Pulsverlauf sein. Zum Bestimmen des Referenzpunktes kann eine Änderungsrate des Pulsverlaufs herangezogen werden. Mit anderen Worten wird der Referenzpunkt auf der Grundlage der Änderungsrate des Pulsverlaufs bestimmt. So wird der Referenzpunkt in Abhängigkeit des vorangehend beschriebenen lokalen Minimums und Maximums in dem Pulsverlauf bestimmt werden, die über die Änderungsrate des Pulsverlaufs eindeutig bestimmbar sind.

Alternativ oder zusätzlich kann der Referenzpunkt auf der Grundlage einer Ableitung der Änderungsrate des Pulsverlaufs bestimmt werden. Die Ableitung der Änderungsrate entspricht dabei einer zweiten Ableitung des Pulsverlaufs.

Der Referenzpunkt wird erfindungsgemäß auf der Grundlage eines Wendepunkts in dem Pulsverlauf bestimmt und entspricht dem Wendepunkt in dem Pulsverlauf. Im Allgemeinen wird unter einem Wendepunkt einer Kurve ein Punkt verstanden, an dem die Kurve ihr Krümmungsverhalten ändert. Zum Bestimmen des Wendepunkts kann eine Nullwertbestimmung der zweiten Ableitung des Pulsverlaufs erfolgen, wobei der Wert einer dritten Ableitung des Pulsverlaufs an dem Wendepunkt ungleich null sein muss. Der dem Referenzpunkt entsprechende Wendepunkt weist eine positive Änderungsrate oder Steigung auf, um sicherzustellen, dass der Referenzpunkt innerhalb des sich zwischen dem lokalen Minimum und dem lokalen Maximum erstreckenden Übergangsbereichs liegt.

Im Folgenden wird der Schritt des Bestimmens des Blutdruckwerts auf der Grundlage des Referenzpunkts näher erläutert.

Der Schritt des Bestimmens des Blutdruckwerts erfolgt auf der Grundlage eines Zeitpunktes und einer Amplitude des Referenzpunktes. So erfolgt der Schritt des Bestimmens des Blutdruckwerts auf der Grundlage eines aus dem Zeitpunkt und der Amplitude gebildeten Quotienten.

Das Bestimmen des Blutdrucks erfolgt mithilfe eines mathematischen Modells oder einer mathematischen Funktion. Entsprechend umfasst das Verfahren ferner einen Schritt des Bereitstellens des mathematischen Modells oder der mathematischen Funktion.

Das mathematische Modell oder die mathematische Funktion ist dazu eingerichtet, in Abhängigkeit wenigstens einer Eingangsgröße, umfassend den Zeitpunkt und/oder die Amplitude des Referenzpunktes, den Blutdruckwert als Ausgangsgröße zu bestimmen. Entsprechend kann der Blutdruckwert als Funktion des Zeitpunktes und der Amplitude des Referenzpunktes nach wenigstens einer der folgenden Gleichungen ausgedrückt werden:

$$BD_n = F_1(t_{RP\_n}), \tag{1}$$

$$BD_n = F_2(h_{RP\_n}), \tag{2}$$

$$BD_n = F_3(t_{RP\_n}; h_{RP\_n}), \tag{3}$$

wobei $BD_n$ den Blutdruckwert für den Herzzyklus n anzeigt; $F$ sich jeweils auf ein mathematisches Modell oder eine mathematische Funktion bezieht; $t_{RP\_n}$ den Zeitpunkt des Referenzpunktes $RP$ für den Herzzyklus n anzeigt; und $h_{RP\_n}$ die Amplitudenhöhe des Referenzpunktes RP für den Herzzyklus n anzeigt.

Wie vorangehend beschrieben erfolgt der Schritt des Bestimmens des Blutdruckwerts auf der Grundlage eines aus dem Zeitpunkt und der Amplitude gebildeten Quotienten. Entsprechend kann der Blutdruckwert als Funktion eines aus dem Zeitpunkt und der Amplitude gebildeten Quotienten wie folgt ausgedrückt werden:

$$BD_n = F\left(\frac{t_{RP\_n}}{h_{RP\_n}}\right) \tag{4}$$

Die mathematische Funktion kann insbesondere eine lineare Funktion sein. Entsprechend können die obigen Gleichungen (1), (2) und (4) wie folgt ausgedrückt werden:

$$BD_n = c_1 + m_1 \times t_{RP\_n}, \tag{5}$$

$$BD_n = c + m_2 \times h_{RP\_n}, \tag{6}$$

$$BD_n = c + m_3 \times \frac{t_{RP\_n}}{h_{RP\_n}}, \tag{7}$$

wobei c jeweils eine Verschiebungskonstante der linearen Funktionen anzeigt; und m jeweils eine Steigung der linearen Funktionen anzeigt.

Alternativ kann die mathematische Funktion eine Polynomfunktion sein, insbesondere eine Polynomfunktion zweiten Grades oder eines höheren Grades.

Der Schritt des Bereitstellens der mathematischen Funktion kann insbesondere dafür vorgesehen sein, auf der Grundlage von gemessenen Werten für den Blutdruckwert und dazugehörigen Pulsverläufen die mathematische Funktion oder das mathematische Modell herzuleiten oder eine bestehende mathematische Funktion oder ein bestehendes mathematisches Modell anzupassen. Eine Anpassung der mathematischen Funktion oder des mathematischen Modells kann entsprechend auch als Kalibrierung bezeichnet werden.

Genauer kann der Schritt des Bereitstellens der mathematischen Funktion einen Teilschritt des Bereitstellens wenigstens zweier Datensätze umfassen. Die Datensätze können jeweils einen gemessenen Blutdruckwert und einen dazugehörigen Zeitpunkt und/oder eine dazugehörige Amplitude des Referenzpunktes aufweisen. Der gemessene Blutdruckwert kann beispielsweise mittels eines Manschetten-basierten, nicht-invasiven Blutdruckmonitors oszillometrisch gemessen und zu einem ermittelten Referenzpunkt zugeordnet werden. Der dazugehörige Referenzpunkt kann insbesondere auf der Grundlage eines zeitlich an die Messung des Blutdruckwerts gekoppelten Pulsverlaufs erfolgen. Der gemessene Blutdruckwert und der dazugehörige Referenzpunkt bilden entsprechend einen voranstehend beschriebenen Datensatz und können als solcher zusammengelegt werden.

In einem weiteren Teilschritt kann dann auf der Grundlage der bereitgestellten Datensätze die mathematische Funktion bestimmt oder hergeleitet werden. Das Bestimmen oder Herleiten der mathematischen Funktion auf der Grundlage der bereitgestellten Datensätze kann insbesondere mittels Interpolation oder Regression, insbesondere linearer Regression, oder mittels eines beliebigen Verfahrens zum Bestimmen einer Polynomfunktion auf der Grundlage von zugehörigen Datensätzen erfolgen. Derartige Verfahren zum Bestimmen oder Herleiten einer mathematischen Funktion auf der Grundlage von zugehörigen Datensätzen sind dem Fachmann bekannt und werden deshalb in der vorliegenden Offenbarung nicht genauer erläutert.

In einer Weiterentwicklung kann das Verfahren einen Schritt des Ausgebens eines Warnsignals umfassen. In dem Schritt kann ein Ausgeben des Warnsignals dann erfolgen, wenn der ermittelte Blutdruckwert einen vorgegebenen Grenzwert erreicht. Der vorgegebene Grenzwert kann in Bezug auf einen Blutdruckwert eines vorausgehenden Herzzyklus, insbesondere zu Beginn einer medizinischen Behandlung, beispielsweise einer Dialysebehandlung, festgelegt sein. In dem Fall, dass der zu bestimmende Blutdruckwert ein systolischer Blutdruckwert ist, kann der Grenzwert durch Subtraktion des Blutdruckwerts eines vorausgehenden Herzzyklus mit einem Wert von im Wesentlichen 15 oder 20 mmHg festgelegt sein.

In dem Fall, dass der zu bestimmende Blutdruckwert ein diastolischer Blutdruckwert ist, kann der Grenzwert durch Subtraktion des Blutdruckwerts eines vorausgehenden Herzzyklus mit einem Wert von im Wesentlichen 15 oder 20 mmHg festgelegt sein.

In dem Fall, dass der zu bestimmende Blutdruckwert einem mittleren arteriellen Druckwert entspricht, kann der Grenzwert durch Subtraktion des Blutdruckwerts eines vorausgehenden Herzzyklus mit einem Wert von im Wesentlichen 7,5 oder 10 mmHg festgelegt sein.

Weiterhin wird ein Blutdruckmessgerät zum Bestimmen eines Blutdrucks eines Patienten vorgeschlagen. Das Blutdruckmessgerät kann dafür vorgesehen sein, das vorangehend beschriebene Verfahren durchzuführen. Die im Zusammenhang mit dem voranstehenden Verfahren beschriebenen technischen Merkmale gelten somit entsprechend für das Blutdruckmessgerät als offenbart, und umgekehrt.

Das Blutdruckmessgerät umfasst eine Messeinheit zum Ermitteln eines Pulsverlaufs des Patienten. Ferner umfasst das Blutdruckmessgerät eine Steuereinheit zum Bestimmen eines Referenzpunktes im Pulsverlauf in einem Übergangsbereich von der Systole zur Diastole eines Herzzyklus des Patienten, und zum Bestimmen des Blutdruckwerts auf der Grundlage des bestimmten Referenzpunktes.

Das Blutdruckmessgerät kann zum Messen eines Blutdruckwertes während einer Dialysebehandlung vorgesehen sein, ist jedoch nicht auf diese Anwendung beschränkt. Das Blutdruckmessgerät kann insbesondere in einem Dialysesystem umfasst sein und im kommunikativen Austausch mit einem Dialysegerät stehen. Entsprechend kann das Blutdruckmessgerät eine Schnittstelle zur Kommunikation mit einem Dialysegerät oder einem anderen Gerät zum Austausch, insbesondere beidseitigen Austausch, von Informationen, insbesondere von gemessenen oder bestimmten Werten, umfassen.

Der grundlegende Aufbau und die grundlegende Funktion eines Dialysegeräts oder -systems sind dem Fachmann bekannt und werden in der vorliegenden Offenbarung nicht näher erläutert. Vielmehr wird auf technische Merkmale des Blutdruckmessgeräts eingegangen, die in Verbindung mit der vorliegenden Erfindung stehen.

Die Messeinheit des Blutdruckmessgeräts kann dafür vorgesehen sein, den Pulsverlauf auf der Grundlage von zeit- und volumenabhängigen Durchblutungswerten an einem Gewebeabschnitt des Patienten zu erfassen, insbesondere mittels Photoplethysmographie. Die Messeinheit kann daher eine photoplethysmografische Messeinheit sein, insbesondere ein Pulsoximeter. Die Messeinheit kann ferner dafür vorgesehen sein, lösbar an einem gut durchbluteten Gewebeabschnitt des Patienten befestigt zu werden. Beispielsweise kann die Messeinheit dafür vorgesehen sein, an einem Arm, insbesondere Unterarm, einem Finger, einem Zeh und/oder an einem Ohr eines Patienten befestigt zu werden. Entsprechend kann die Messeinheit ein Armband, einen Fingerclip, einen Zehclip und/oder einen Ohrclip aufweisen.

Das Blutdruckmessgerät kann zum kontinuierlichen Überwachen eines Pulsverlaufs des Patienten vorgesehen sein. Aufbauend darauf kann das Blutdruckmessgerät für jeden während der Überwachung aufgenommen Herzzyklus wenigstens einen Blutdruckwert bestimmen. Dies erfolgt auf der Grundlage des durch die Steuereinheit bestimmten Referenzpunktes, der jeweils für einen aufgenommenen Herzzyklus des Patienten bestimmt werden kann. Auf diese Weise kann eine durchgehende Überwachung des Blutdruckwerts, insbesondere während einer medizinischen Behandlung, sichergestellt werden.

Zum Bestimmen des Blutdruckwerts auf der Grundlage des bestimmten Referenzpunktes kann sich das Blutdruckmessgerät eines mathematischen Modells oder einer mathematischen Funktion bedienen, die auf der Grundlage des Referenzpunktes als Eingangsgröße den Blutdruckwert als Ausgangsgröße ausgibt. Das mathematische Modell oder die mathematische Funktion kann in einer Speichereinheit des Blutdruckmessgeräts abgelegt sein. Dabei kann die Steuereinheit Zugriff auf die Speichereinheit und das darin gespeicherte mathematische Modell oder die darin gespeicherte mathematische Funktion haben, um den Blutdruckwert auf der Grundlage des bestimmten Referenzpunktes zu berechnen.

In einer Weiterentwicklung kann die Steuereinheit dazu eingerichtet sein, die in der Speichereinheit abzulegende Funktion zu bestimmen und/oder eine in der Speichereinheit abgelegte Funktion anzupassen. Dies kann auf der Grundlage von der Steuereinheit bereitgestellten Messdaten oder Datensätzen erfolgen. Die Datensätze können jeweils einen gemessenen Blutdruckwert und einen dazugehörigen Referenzpunkt, insbesondere einen dazugehörigen Zeitpunkt und/oder eine dazugehörige Amplitude des Referenzpunktes, umfassen. Die Datensätze können patientenspezifisch bereitgestellt sein. Auf diese Weise kann eine Bestimmung von patientenspezifischen mathematischen Modellen oder Funktionen zum Ermitteln des Blutdruckwerts sichergestellt werden. die Datensätze sind vorzugsweise derart bereitgestellt, dass die gemessenen Blutdruckwerte jeweils an einem vordefinierten Körperteil, insbesondere jeweils an demselben Körperteil des Patienten erfasst wurden.

In einer Ausführungsform können der Steuereinheit des Blutdruckmessgeräts an dem Patienten gemessenen Blutdruckwerte über die Schnittstelle übermittelt werden. Aufbauend darauf kann die Steuereinheit dazu eingerichtet sein, zu den gemessenen Blutdruckwerten jeweils einen Pulsverlauf eines Herzzyklus und/oder einen Referenzpunkt in dem Pulsverlauf zuzuordnen, um wenigstens zwei Datensätze zu erzeugen, in denen insbesondere gemessenen Blutdruckwerte zu entsprechenden Referenzpunkten zugeordnet sind. Auf der Grundlage der derart ermittelten Datensätze kann die Steuereinheit dann die mathematische Funktion bestimmen, insbesondere mittels eines Interpolation- oder Regressionsverfahrens, beispielsweise eines linearen Regressionsverfahrens.

Alternativ können bereits erstellte Datensätze dem Blutdruckmessgerät über die Schnittstelle übermittelt werden. Beispielsweise können die Datensätze durch ein mit dem Blutdruckmessgerät in Verbindung stehendes Gerät, beispielsweise dem Dialysegerät, erzeugt werden. Hierbei kann das Blutdruckmessgerät über die Schnittstelle den zeitabhängigen Pulsverlauf an dieses Gerät übertragen. Auch kann die mathematische Funktion als solche dem Blutdruckmessgerät übergeben und in der Speichereinheit abgelegt werden. Entsprechend kann ein Bereitstellen oder Herleiten der mathematischen Funktion in dem mit dem Blutdruckmessgerät in Verbindung stehenden Gerät erfolgen.

Zum Erzeugen der gemessenen Blutdruckwerte kann das mit dem Blutdruckmessgerät in Verbindung stehende Gerät, beispielsweise das Dialysegerät, ein weiteres Blutdruckmessgerät umfassen, das zum Messen des Blutdrucks vorgesehen ist. Das weitere Blutdruckmessgerät kann beispielsweise ein Manschetten-basierter, nicht-invasiver Blutdruckmonitor sein, der den Blutdruck eines Patienten oszillometrisch in regelmäßigen Abständen, beispielsweise im 10-Minuten-Takt, misst.

In einer Weiterentwicklung kann das Blutdruckmessgerät eine Warnsignaleinheit umfassen, die dazu eingerichtet sein kann, ein Warnsignal auszugeben, wenn der durch die Steuereinheit ermittelte Blutdruckwert einen vorgegebenen Grenzwert erreicht. Das Warnsignal kann ein optisches Signal und/oder ein akustisches Signal sein, insbesondere ein Alarmsignal. Insbesondere kann der vorgegebene Grenzwert in Bezug auf einen Blutdruckwert eines vorausgehenden Herzzyklus, beispielsweise zu Beginn einer medizinischen Behandlung oder der Dialysebehandlung festgelegt sein. Auf diese Weise kann das Blutdruckmessgerät beispielsweise eine während einer Dialysebehandlung auftretende Intradialytische Hypotension anzeigen.

Entsprechend kann das vorgeschlagene Blutdruckmessgerät zur Diagnose einer Intradialytischen Hypotension eingerichtet sein. Das Dialysegerät kann dabei in Erwiderung auf das durch das Blutdruckmessgerät ausgegebene Warnsignal einem weiteren Abfall im Blutdruck des Patienten entgegenwirken. Hierzu kann das Dialysegerät eine Ultrafiltration und somit einen weiteren Wasserentzug aus dem Blut des Patienten stoppen. Alternativ oder zusätzlich kann das Dialysegerät bewirken, dass der Patient eine stabile Position, beispielsweise in die sogenannte Tendelenburg-Position, positioniert wird. Alternativ oder zusätzlich kann in Erwiderung auf das Warnsignal, medizinisches Personal informiert werden und oder eine Blutdruckmessung zum Validieren des Blutdruckabfalls initiiert werden.

Weiterhin wird ein Dialysesystem vorgeschlagen, dass ein vorangehend beschriebenes Blutdruckmessgerät umfasst. Die in Verbindung mit dem Blutdruckmessgerät vorangehend beschriebenen technischen Merkmale gelten somit auch für das Dialysesystem als offenbart. Das Dialysesystem umfasst ferner vorzugsweise ein Dialysegerät, insbesondere zum Durchführen einer Hämodialyse, Hämofiltration oder Hämodiafiltration.

Kurze Beschreibung der Figuren

Bevorzugte weitere Ausführungsformen der Erfindung werden durch die nachfolgende Beschreibung der Figuren näher erläutert. Dabei zeigen:

Figur 1    ein Dialysesystem umfassend ein Blutdruckmessgerät zum Bestimmen eines Blutdruckwerts eines Patienten auf der Grundlage eines gemessenen Pulsverlaufs;

Figur 2    ein Diagramm, in dem der durch das Blutdruckmessgerät gemessene Pulsverlauf veranschaulicht ist;

Figur 3    ein Flussdiagramm, das ein durch das Blutdruckmessgerät durchgeführtes Verfahren zum Bestimmen des Blutdruckwerts des Patienten veranschaulicht;

Figur 4    ein Diagramm, das einen normierten Pulsverlauf eines Herzzyklus des Patienten veranschaulicht;

Figur 5    ein Diagramm, in dem eine Korrelation zwischen gemessenen Blutdruckwerten und ermittelten Referenzpunkten veranschaulicht ist; und

Figur 6    ein Diagramm, in dem ein durchschnittlicher Verlauf des systolischen Blutdrucks eines Patienten während einer Hämodialyse veranschaulicht ist.

Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

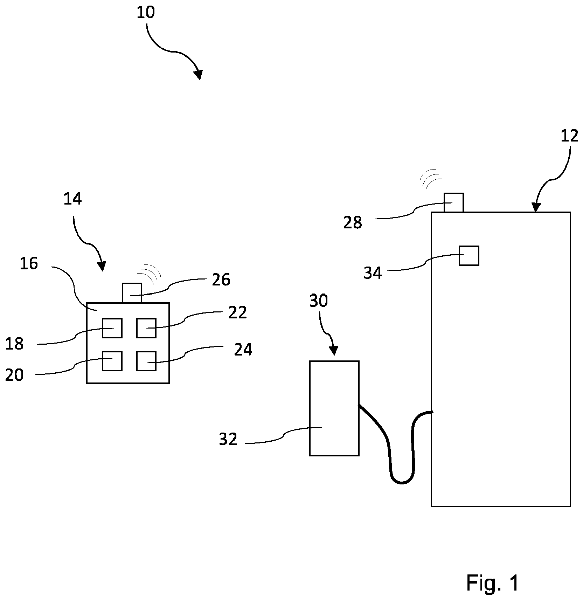

Figur 1 zeigt schematisch ein Dialysesystem 10 zur Durchführung einer Hämodialyse an einem Patienten. Das Dialysesystem 10 umfasst ein Dialysegerät 12 und ein Blutdruckmessgerät 14 zum Bestimmen eines Blutdruckwerts eines Patienten auf der Grundlage eines gemessenen Pulsverlaufs.

Das Blutdruckmessgerät 14 umfasst ein Armband 16, das während der Dialysebehandlung lösbar an einen Unterarm oder Oberarm des Patienten befestigt werden kann. Das Armband 16 ist mit einer Messeinheit 18, einer Steuereinheit 20, einer Speichereinheit 22, einer Warnsignaleinheit 24 und einer Funkschnittstelle 26 zum Datenaustausch mit einer dazu komplementären Funkschnittstelle 28 an dem Dialysegerät 12 versehen.

Die Messeinheit 18 ist dazu eingerichtet, einen zeitabhängigen Pulsverlauf des Patienten zu messen. Die Messeinheit 18 ist zur nicht-invasiven Messung des Pulses vorgesehen. Beispielsweise kann die Messeinheit 18 eine photoplethysmografische Messeinheit sein, die mittels Photoplethysmographie zeit- und volumenabhängige Durchblutungswerte an einem Gewebeabschnitt des Patienten misst und aufbauend darauf den Pulsverlauf des Patienten bestimmt.

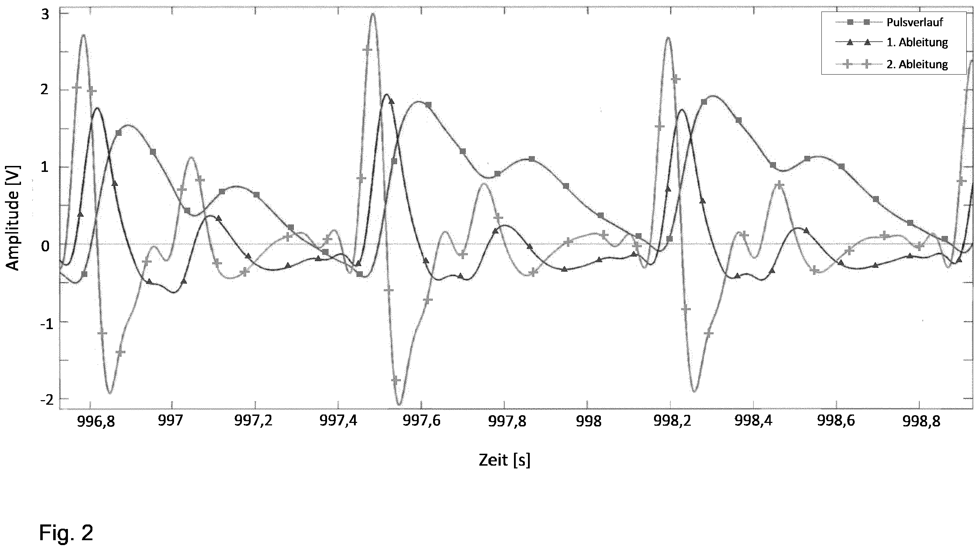

Ein mittels der Messeinheit 18 bestimmter Pulsverlauf des Patienten ist in dem in Figur 2 gezeigten Diagramm veranschaulicht, in dem eine Amplitude eines Pulses des Patienten über die Zeitdauer von drei Herzzyklen abgebildet ist. Die Abszisse des Diagramms zeigt die Zeitdauer und die Ordinate des Diagramms die Amplitude eines durch die Messeinheit 18 erfassten Signals.

Die Steuereinheit 20 ist zur Verarbeitung der durch die Messeinheit 18 gemessenen Daten eingerichtet. Genauer ist die Steuereinheit 20 dazu eingerichtet, einen Referenzpunkt RP in dem Pulsverlauf in einem Übergangsbereich ÜB von der Systole zur Diastole eines Herzzyklus des Patienten zu bestimmten und einen Blutdruckwert des Patienten auf der Grundlage des bestimmten Referenzpunktes zu ermitteln.

Alternativ oder zusätzlich zu der Anordnung der photoplethysmografischen Messeinheit in dem hier beschriebenen Armband kann die photoplethysmografische Messeinheit auch in einem Ohrclip, Fingerclip oder Zehenclip angeordnet sein.

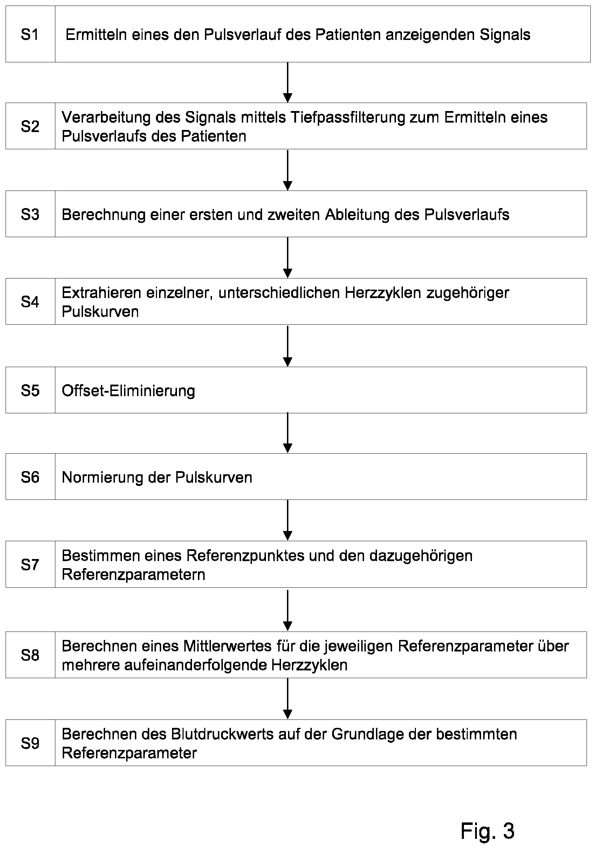

Im Folgenden wird ein durch die Steuereinheit 20 des Blutdruckmessgeräts 14 durchgeführtes Verfahren zum Bestimmen von Blutdruckwerten unter Bezugnahme auf Figur 3 genauer erläutert.

In einem ersten Schritt S1 des Verfahrens wird ein den Pulsverlauf des Patienten anzeigendes Signal mittels der Messeinheit 18 ermittelt und an die Steuereinheit 20 übertragen. Zur Eliminierung von Hochfrequenzstörungen führt die Steuereinheit 20 in einem Schritt S2 zunächst eine Tiefpassfilterung, beispielsweise für einen Frequenzbereich von 20 Hz, durch, um den in Figur 2 gezeigten Pulsverlauf zu ermitteln. Darauffolgend berechnet die Steuereinheit 20 in einem Schritt S3 eine erste und zweite Ableitung des Pulsverlaufs, die in Figur 2 veranschaulicht sind und die zum Bestimmen des Referenzpunktes RP herangezogen werden.

In einem weiteren Schritt S4 ist die Steuereinheit 20 dazu eingerichtet, unterschiedliche Herzzyklen oder unterschiedlichen Herzzyklen zugeordnete Pulskurven in dem Pulsverlauf zu ermitteln und zur Weiterverarbeitung zu extrahieren. Hierzu kann die Steuereinheit 20 dazu eingerichtet sein, jeweils einen Anfangs- und einen Endpunkt je Pulskurve zu bestimmen.

Die darauffolgenden Schritte S5 bis S7 werden dann für jede erkannte Pulskurve oder für den Pulsverlauf eines jeden erkannten Herzzyklus durchgeführt.

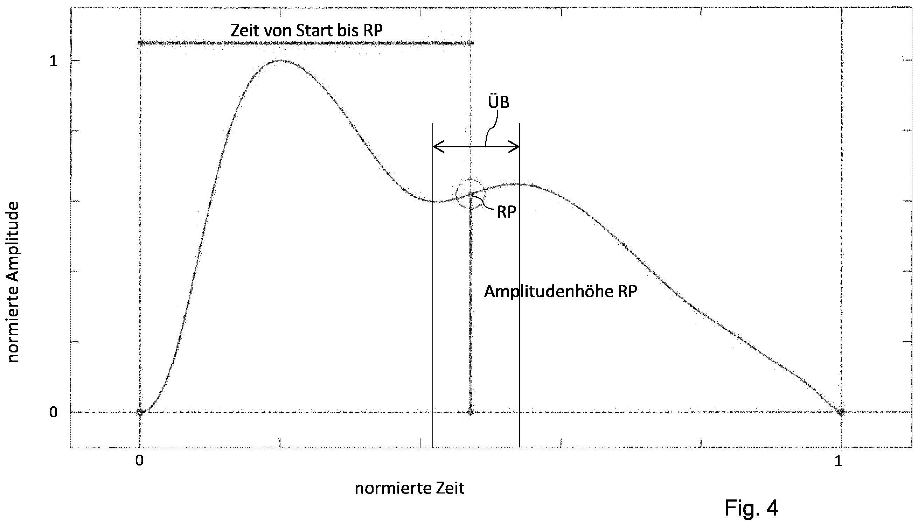

In dem Schritt S5 erfolgt zunächst eine Offset-Eliminierung für jede einzelne Pulskurve, bevor in dem Schritt S6 eine amplituden- und zeitabhängige Normierung der einzelnen Pulskurven erfolgt. Hierzu wird zunächst eine maximale Amplitude und eine Herzzyklusdauer oder Herzperiode für jede einzelne Pulskurve bestimmt. Darauffolgend wird die Amplitude einer einzelnen Pulskurve durch das zugehörige Maximum und die Breite der Pulskurve durch die zugehörige Herzzyklusdauer normiert, um für jeden der erkannten Herzzyklen jeweils einen wie in Figur 4 veranschaulichten normierten Pulsverlauf zu ermitteln. Genauer zeigt Figur 4 einen normierten Pulsverlauf für einen Herzzyklus, wobei die Abszisse normierte Zeitdauerwerte und die Ordinate normierte Amplitudenwerte anzeigen.

In einer Weiterentwicklung kann eine patientenabhängige Parameterbestimmung durchgeführt werden. Die heranzuziehenden patientenspezifischen Parameter können hierbei an die Steuereinheit 20 übergeben werden, beispielsweise aus einer Patientenakte oder einer Patientenkarte, die insbesondere mithilfe des Dialysegeräts 12 erfasst und an die Steuereinheit 20 übermittelt werden können.

Ausgehend von dem normierten Pulsverlauf wird daraufhin in dem Schritt S7 ein Referenzpunkt RP im Pulsverlauf für jeden Herzzyklus bestimmt. Vorliegend betrifft der Referenzpunkt RP einen mathematisch eindeutig bestimmbaren Punkt im Pulsverlauf in einem Übergangsbereich ÜB von der Systole zur Diastole eines Herzzyklus des Patienten. Der Übergangsbereich ist in Figur 4 veranschaulicht und erstreckt sich zwischen einem lokalen Minimum und einem lokalen Maximum in dem Pulsverlauf. Genauer entspricht der Referenzpunkt RP einem Wendepunkt in dem Pulsverlauf, der eine positive Änderungsrate oder Steigung aufweist. Zum Bestimmen des Wendepunkts zieht die Steuereinheit 20 die ermittelte zweite Ableitung des Pulsverlaufs heran. Mit anderen Worten bestimmt die Steuereinheit 20 den Referenzpunkt auf der Grundlage der zweiten Ableitung des Pulsverlaufs. Im Speziellen kann die Steuereinheit 20 dazu eingerichtet sein, einen Punkt zwischen dem lokalen Minimum und dem lokalen Maximum zu bestimmen, für den die zweite Ableitung den Wert Null annimmt.

Für den bestimmten Referenzpunkt RP werden daraufhin ein Zeitpunkt des Referenzpunktes, eine Amplitudenhöhe und ein daraus gebildeter Quotient für jeden der identifizierten Herzzyklen ermittelt, die nachfolgend als Referenzparameter bezeichnet werden.

In einem Schritt S8 wird dann ein Mittelwert für die Referenzparameter für die ermittelten, aufeinanderfolgenden Herzzyklen bestimmt. Dies kann in dem Verfahren fortlaufend erfolgen. Beispielsweise kann die Steuereinheit 20 dazu eingerichtet sein, einen Mittelwert für die Referenzparameter auf der Grundlage von drei Herzzyklen zu bestimmen.

In einem nächsten Schritt S9 bestimmt die Steuereinheit 20 einen systolischen Blutdruckwert auf der Grundlage wenigstens einer der nachfolgenden drei linearen Funktionen:

$$SBP = c + m_1 \times \bar{t} \qquad\qquad (8)$$

$$SBP = c_2 + m_2 \times \bar{h}, \qquad\qquad (9)$$

$$SBP = c + m_3 \times \frac{\bar{t}}{\bar{h}}, \qquad\qquad (10)$$

wobei *SBP* den systolischen Blutdruckwert anzeigt ; c jeweils eine Verschiebungskonstante der linearen Funktionen anzeigt; m jeweils eine Steigerung der linearen Funktionen anzeigt; $\bar{t}$ den gemittelten Zeitpunkt des Referenzpunktes beispielsweise über zwei bis 20 Herzzyklen, bevorzugt 5 bis 20 Herzzyklen anzeigt; und $\bar{h}$ die gemittelte Amplitudenhöhe des Referenzpunktes beispielsweise über zwei bis 20 Herzzyklen, bevorzugt 5 bis 20 Herzzyklen anzeigt .

Dabei kann auch mit einem gleitenden Mittelwert gearbeitet werden, bei dem eine Fenstergröße für den jeweils gewählten Parameter je nach gewünschter oder geforderter Genauigkeit des Verfahrens gewählt wird.

Alternativ kann die Steuereinheit 20 in dem Schritt S9 einen mittleren arteriellen Blutdruckwert auf der Grundlage der bestimmten Referenzparameter mithilfe einer mathematischen Funktion bestimmen.

Die linearen Funktionen sind vorzugsweise in der Speichereinheit 22 des Blutdruckmessgeräts 14 gespeichert. Alternativ zu den oben genannten Funktionen kann zumindest eine Polynomfunktion zweiten Grades oder eines höheren Grades zum Bestimmen des Blutdruckwerts verwendet werden.

In dem hier gezeigten Dialysesystem 10 ist das Dialysegerät 12 dazu eingerichtet, die vorangehend beschriebenen Funktionen zum Berechnen des Blutdruckwerts des Patienten herzuleiten und/oder während des Betriebs anzupassen. Alternativ kann die Steuereinheit 20 des Blutdruckmessgeräts 14 dazu eingerichtet sein, die vorangehend beschriebenen Funktionen zum Berechnen des Blutdruckwerts des Patienten herzuleiten und/oder während des Betriebs anzupassen. Dies erfolgt auf der Grundlage von dem Dialysegerät bereitgestellten Datensätzen, in denen jeweils ein gemessener Blutdruckwert mit einem zugehörigen Referenzpunkt, insbesondere den zugehörigen Referenzparametern, enthalten sind.

Zum Erzeugen der gemessenen Blutdruckwerte umfasst das Dialysegerät 12 ein weiteres Blutdruckmessgerät 30. Genauer ist das weitere Blutdruckmessgerät 30 als ein Manschetten-basierter, nicht-invasiver Blutdruckmonitor bereitgestellt, der den Blutdruck eines Patienten oszillometrisch in regelmäßigen Abständen, beispielsweise im 10-Minuten-Takt oder stündlich, während der Dialysebehandlung misst. Hierzu wird an dem Arm des Patienten eine Manschette 32 befestigt, mittels derer ein systolischer und/oder diastolischer Blutdruckwert des Patienten bestimmt wird.

Das Dialysegerät 12 ist dazu eingerichtet, den gemessenen Blutdruckwerten jeweils einen zugehörigen Referenzpunkt und entsprechend die zugehörigen Referenzparameter des Referenzpunktes zuzuordnen, um so jeweils einen Datensatz zu erzeugen. Hierzu ist die Steuereinheit 20 dazu eingerichtet, der Dialyseeinheit 12 zu den jeweiligen Zeitpunkten der durchgeführten Blutdruckmessungen mittels des weiteren Blutdruckmessgerät 30 die zugehörigen Referenzparameter zu übermitteln. Die derart erzeugten Datensätze werden in einer weiteren Speichereinheit 34 in dem Dialysegerät 12 abgespeichert.

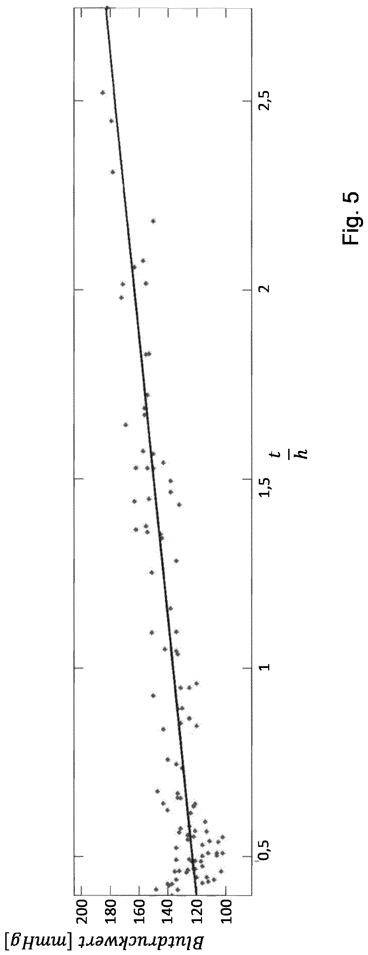

Figur 5 zeigt ein Diagramm, in dem derart erzeugte Datensätze veranschaulicht sind. Die Datensätze umfassen einen gemessenen Blutdruckwert und einen dazugehörigen ermittelten Referenzquotienten, die als Punkte in dem Diagramm veranschaulicht sind. Der Referenzquotient setzt einen Zeitpunkt zu einer Amplitudenhöhe eines Referenzpunktes ins Verhältnis. Entsprechend zeigt die Abszisse einen Wert für den Referenzquotienten und die Ordinate einen Wert für den gemessenen Blutdruckwert an.

Auf der Grundlage der in der weiteren Speichereinheit 34 gespeicherten Datensätze ist die Dialyseeinheit 12 dazu eingerichtet, die mathematischen Funktionen, insbesondere die Koeffizienten c und $m_{1\text{-}3}$ der obigen Gleichungen (8), (9) und (10) mittels linearer Regression zu bestimmen. Im Hinblick auf die Gleichung (10) ist das Ergebnis dieses Verfahrensschritts als lineare Funktion in dem in Figur 5 gezeigten Diagramm veranschaulicht.

Nach erfolgter Bestimmung oder Anpassung der mathematischen Funktionen, werden die Koeffizienten c und $m_{1\text{-}3}$ der obigen Gleichungen (8), (9) und (10) über die Funkschnittstellen 26 und 28 an das Blutdruckmessgerät 14 übermittelt und in dessen Speichereinheit 22 abgelegt. Auf diese Weise wird sichergestellt, dass die Steuereinheit 20 die Blutdruckwerte auf der Grundlage der durch das Dialysegerät 12 ermittelten oder angepassten mathematischen Funktionen berechnet.

Das Dialysegerät 12 ist dazu eingerichtet, in regelmäßigen und vordefinierten Abständen, beispielsweise alle drei oder vier Wochen, eine Kalibrierung oder Anpassung der mathematischen Funktionen, insbesondere der Koeffizienten $c_{1\text{-}3}$ und $m_{1\text{-}3}$ durchzuführen. Weiterhin ist das Dialysegerät 12 dazu eingerichtet, die ermittelten Koeffizienten $c_{1\text{-}3}$ und $m_{1\text{-}3}$, vor Beginn einer Dialysebehandlung an das Blutdruckmessgerät 14 zu übermitteln.

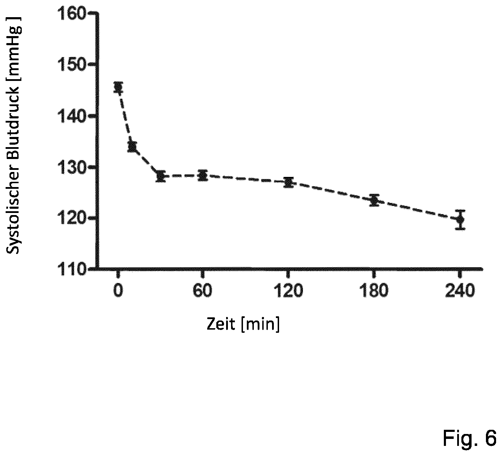

Alternativ oder zusätzlich kann auch ein Anpassen und Bestimmen der mathematischen Funktionen und deren Übertragen an das Blutdruckmessgerät 14 während einer Dialysebehandlung erfolgen. Figur 6 zeigt ein Diagramm, das einen durchschnittlichen Verlauf des systolischen Blutdrucks eines Patienten während einer Hämodialyse veranschaulicht. Die Abszisse des Diagramms zeigt den Zeitverlauf und die Ordinate den systolischen Blutdruck. Daraus geht hervor, dass in den ersten 30 Minuten der Dialysebehandlung der systolische Blutdruck besonders stark abfällt. Dies bietet die Möglichkeit, für die Anpassung oder Herleitung der mathematischen Funktionen geeignete Datensätze zu Beginn der Dialysebehandlung zu erheben und darauf aufbauend ein Anpassen oder Bereitstellen der mathematischen Funktionen durchzuführen, auf deren Grundlage dann im weiteren Verlauf der Behandlung die Bestimmung der Blutdruckwerte erfolgen kann.

In einer alternativen Konfiguration können das Blutdruckmessgerät 14 und das weitere Blutdruckmessgerät 30 in einem einzigen Armband oder einer einzigen Manschette angeordnet sein. Auf diese Weise kann sichergestellt werden, dass beide Blutdruckmessgeräte an derselben Körperstelle des Patienten Messungen durchführen. Dadurch kann der Komfort für einen Patienten verbessert werden. Das Blutdruckmessgerät 14 kann dabei dazu eingerichtet sein, vor und nach einer durch das weitere Blutdruckmessgerät 30 durchgeführten Messung jeweils einen Blutdruckwert zu ermitteln.

Das Blutdruckmessgerät 14 umfasst ferner die Warnsignaleinheit 24 zur Ausgabe eines Warnsignals. Die Warnsignaleinheit 24 ist dazu eingerichtet, das Warnsignal auszugeben, wenn der durch die Steuereinheit 20 ermittelte Blutdruckwert einen vorgegebenen Grenzwert erreicht oder unterschreitet. Genauer ist die Warnsignaleinheit 24 dazu eingerichtet, das Warnsignal auszugeben, wenn die Steuereinheit 20 bestimmt, dass im Verlauf der Dialysebehandlung ein durch die Steuereinheit 20 bestimmter Blutdruckmesswert einen zu Beginn der Dialysebehandlung bestimmten oder gemessenen Wert um wenigstens 15 oder 20 mmHg unterschreitet. Mit anderen Worten, in dieser Ausgestaltung bezieht sich der Grenzwert auf eine absolute Änderung des bestimmten Blutdruckmesswerts bezogen auf den Messwert zu Beginn der Dialysebehandlung. Alternativ kann sich der Grenzwert auch auf eine relative Änderung des bestimmten Blutdruckmesswerts bezogen auf den Messwert zu Beginn der Dialysebehandlung beziehen.

Die Warnsignaleinheit 24 ist dazu eingerichtet, in Erwiderung auf das erzeugte Warnsignal, ein optischen und ein akustischen Alarm auszugeben. Weiterhin ist die Warnsignaleinheit 24 dazu eingerichtet, das erzeugte Warnsignal über die Funkschnittstelle 26 an das Dialysegerät 12 zu übermitteln. In Erwiderung auf das Warnsignal kann das Dialysegerät 12 dazu eingerichtet sein, eine Blutdruckmessung mittels des weiteren Blutdruckmessgeräts 30 auszuführen. Alternativ oder zusätzlich kann das Dialysegerät in Erwiderung auf das Warnsignal eine Veränderung einer Position des Patienten, insbesondere in die sogenannte Tendelenburg-Position, veranlassen. Alternativ oder zusätzlich kann das Dialysegerät in Erwiderung auf das Warnsignal medizinisches Behandlungspersonal anfordern. Alternativ oder zusätzlich kann das Dialysegerät 12 in Erwiderung auf das Warnsignal ein Ultrafiltrationsverfahren des Blutes des Patienten unterbrechen.

In einer Weiterentwicklung kann die Warnsignaleinheit 24 dazu eingerichtet sein, ein zweistufiges Warnsignal auszugeben. In dieser Konfiguration kann die Warnsignaleinheit 24 ein erstes Warnsignal ausgeben, wenn der bestimmte Blutdruckmesswert den zu Beginn der Dialysebehandlung bestimmten oder gemessenen Blutdruckwert um einen Wert zwischen 15 und 20 mmHg unterschreitet. Weiterhin kann die Warnsignaleinheit 24 ein zweites Warnsignal ausgeben, wenn der während des Dialyseverfahrens bestimmte Blutdruckmesswert den zu Beginn der Dialysebehandlung bestimmten oder gemessenen Blutdruckwert um wenigstens 20 mmHg unterschreitet.

In einer Ausführungsform kann das Dialysegerät 12 dazu eingerichtet sein, auf Erwiderung auf das Warnsignal zunächst eine Messung mit dem weiteren Blutdruckmessgerät 30 durchzuführen. Nur wenn auch diese Messung ergibt, dass der gemessene Blutdruckwert den Grenzwert erreicht hat, wird ein Alarmsignal, insbesondere an das medizinische Behandlungspersonal, ausgegeben und/oder die Ultrafiltration unterbrochen und/oder eine Veränderung der Position des Patienten veranlasst.

In einer Weiterentwicklung kann das Dialysegerät 12 dazu eingerichtet sein, auf der Grundlage der mittels der Blutdruckmesseinheit 14 bestimmten und an diese über die Funkschnittstellen 26, 28 übermittelten Blutdruckmesswerte ein durch das Dialysegerät 12 durchgeführtes Ultrafiltrationsverfahren zu steuern. Hierbei können die übermittelten Blutdruckmesswerte als Eingangsgröße für einen geschlossenen Regelkreis zum Regeln einer Ultrafiltrationsrate bei dem Ultrafiltrationsverfahren dienen.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

Bezugszeichenliste

10    Dialysesystem
12    Dialysegerät
14    Blutdruckmessgerät
16    Armband
18    Messeinheit
20    Steuereinheit
22    Speichereinheit
24    Warnsignaleinheit
26    Funkschnittstelle des Blutdruckmessgerät
28    Funkschnittstelle des Dialysegeräts
30    weiteres Blutdruckmessgerät
32    Manschette
34    weitere Speichereinheit

**Patentansprüche**

1.  Verfahren zum Bestimmen eines Blutdruckwerts eines Patienten, umfassend die Schritte:

    - Erfassen einer nicht-invasiven Messung eines Pulses des Patienten und Ermitteln eines Pulsverlaufs des Patienten mittels einer Messeinheit (18);
    - Bestimmen, mittels einer Steuereinheit (20), eines Referenzpunktes (RP) im Pulsverlauf in einem Übergangsbereich (ÜB) von der Systole zur Diastole eines Herzzyklus des Patienten, wobei der Referenzpunkt (RP) ein eine positive Änderungsrate oder Steigung aufweisender Wendepunkt zwischen einem lokalen Minimum und einem lokalen Maximum ist; und
    - Bestimmen, mittels der Steuereinheit (20), des Blutdruckwerts auf der Grundlage des bestimmten Referenzpunktes (RP),

    **dadurch gekennzeichnet, dass** das Bestimmen anhand einer bereitgestellten mathematischen Funktion erfolgt, welche ein aus dem Zeitpunkt (t) und der Amplitude (h) des Referenzpunktes (RP) gebildeten Quotienten als Eingangsgröße zu einem Blutdruckwert als Ausgangsgröße korreliert.

2.  Verfahren nach Anspruch 1, wobei der Blutdruckwert einem systolischen und/oder einem diastolischen und/oder einem mittleren arteriellen Blutdruckwert entspricht.

3.  Verfahren nach Anspruch 1 oder 2, bei dem der Pulsverlauf auf der Grundlage von zeit- und volumenabhängigen Durchblutungswerten an einem Gewebeabschnitt des Patienten erfasst wird, insbesondere mittels Photoplethysmographie.

4. Verfahren nach einem der Ansprüche 1 bis 3, bei dem der Pulsverlauf amplituden- und/oder zeitnormiert, insbesondere herzzyklusdauer-normiert, ist.

5. Verfahren nach einem der vorstehenden Ansprüche, bei dem die mathematische Funktion eine lineare Funktion oder eine Polynomfunktion ist.

6. Verfahren nach einem der vorstehenden Ansprüche, bei dem der Schritt des Bereitstellens der mathematischen Funktion folgende Teilschritte umfasst:

   - Bereitstellen wenigstens zweier Datensätze, umfassend einen gemessenen Blutdruckwert und einen dazugehörigen Zeitpunkt (t) und/oder eine dazugehörige Amplitude (h) eines Referenzpunktes (RP); und
   - Bestimmen der mathematischen Funktion auf der Grundlage der bereitgestellten Datensätze, insbesondere mittels eines Interpolations- oder Regressionsverfahrens.

7. Verfahren nach einem der vorstehenden Ansprüche, ferner umfassend einen Schritt des Ausgebens eines Warnsignals, wenn der ermittelte Blutdruckwert einen vorgegebenen Grenzwert erreicht, wobei insbesondere der vorgegebene Grenzwert in Bezug auf einen Blutdruckwert eines vorausgehenden Herzzyklus, insbesondere zu Beginn einer medizinischen Behandlung, festgelegt ist.

8. Blutdruckmessgerät (14) zum Bestimmen eines Blutdruckwerts eines Patienten, umfassend:

   - eine Messeinheit (18) zum Ermitteln eines Pulsverlaufs des Patienten; und
   - eine Steuereinheit (20) zum Bestimmen eines Referenzpunktes (RP) im Pulsverlauf in einem Übergangsbereich von der Systole zur Diastole eines Herzzyklus des Patienten, und zum Bestimmen des Blutdruckwerts auf der Grundlage des bestimmten Referenzpunktes (RP),

   wobei die Steuereinheit (20) dazu eingerichtet ist, ein eine positive Änderungsrate oder Steigung aufweisender Wendepunkt zwischen einem lokalen Minimum und einem lokalen Maximum als Referenzpunkt (RP) zu bestimmen,
   **dadurch gekennzeichnet, dass** die Steuereinheit dazu eingerichtet ist, den Blutdruckwert weiterhin anhand einer bereitgestellten mathematischen Funktion, welche ein aus dem Zeitpunkt (t) und der Amplitude (h) des Referenzpunktes (RP) gebildeten Quotienten als Eingangsgröße zu einem Blutdruckwert als Ausgangsgröße korreliert, zu bestimmen.

9. Blutdruckmessgerät nach Anspruch 8, ferner umfassend eine Schnittstelle (26), über die der Steuereinheit (20) am Patienten gemessene Blutdruckwerte übermittelt werden, wobei die Steuereinheit (20) dazu eingerichtet ist, auf der Grundlage der gemessenen Blutdruckwerte und einem dazugehörigen Pulsverlauf die mathematische Funktion bereitzustellen oder anzupassen.

10. Blutdruckmessgerät nach Anspruch 8 oder 9, ferner umfassend eine Warnsignaleinheit (24), die dazu eingerichtet ist, ein Warnsignal auszugeben, wenn der durch die Steuereinheit (22) ermittelte Blutdruckwert einen vorgegebenen Grenzwert erreicht, wobei insbesondere der vorgegebene Grenzwert in Bezug auf einen Blutdruckwert eines vorausgehenden Herzzyklus, insbesondere zu Beginn einer medizinischen Behandlung, festgelegt ist.

11. Dialysesystem (10), umfassend ein Blutdruckmessgerät (14) nach einem der Ansprüche 8 bis 10.

## Claims

1. Method for determining a blood pressure value of a patient, comprising the steps:

   - detecting a non-invasive measurement of a pulse of the patient and determining a pulse wave of the patient by means of a measuring unit (18);
   - determining, by means of a controller (20), a reference point (RP) in the pulse wave in a transitional region (ÜB) from the systole to the diastole of a cardiac cycle of the patient, wherein the reference point (RP) is a turning point having a positive rate of change or gradient between a local minimum and a local maximum; and
- determining, by means of the controller (20), the blood pressure value on the basis of the determined reference point (RP),

**characterized in that** the determining is performed based on a provided mathematical function which correlates a quotient formed from the time point (t) and the amplitude (h) of the reference point (RP) as the input variable to a blood pressure value as the output variable.

2. Method according to claim 1, wherein the blood pressure value corresponds to a systolic and/or a diastolic and/or an mean arterial blood pressure value.

3. Method according to claim 1 or 2, wherein the pulse wave is recorded on the basis of time and volume-dependent blood flow values in a tissue section of the patient, in particular by means of photoplethysmography.

4. Method according to any one of claims 1 to 3, wherein the pulse wave is normalized in amplitude and/or time, in particular normalized in cardiac cycle duration.

5. Method according to any one of the preceding claims, wherein the mathematical function is a linear function or a polynomial function.

6. Method according to any one of the preceding claims, wherein the step of providing the mathematical function comprises the following substeps:

    - providing at least two data sets, comprising a measured blood pressure value and an associated time point (t) and/or an associated amplitude (h) of a reference point (RP); and
    - determining the mathematical function on the basis of the provided data sets, in particular by means of an interpolation or regression method.

7. Method according to any one of the preceding claims, further comprising a step of outputting a warning signal, when the determined blood pressure value reaches a predetermined limit value, wherein in particular the predetermined limit value is set with respect to a blood pressure value of a preceding cardiac cycle, in particular at the beginning of a medical treatment.

8. Blood pressure measurement device (14) for determining a blood pressure value of a patient, comprising:

    - a measuring unit (18) for determining a pulse wave of the patient; and
    - a controller (20) for determining a reference point (RP) in the pulse wave in a transition region from the systole to the diastole of a cardiac cycle of the patient, and for determining the blood pressure value on the basis of the determined reference point (RP), wherein the controller (20) is configured to determine a turning point as a reference point (RP) that has a positive rate of change or gradient between a local minimum and a local maximum,

    **characterized in that** the control unit is configured to determine the blood pressure value further based on a provided mathematical function which correlates a quotient formed from the time point (t) and amplitude (h) of the reference point (RP) as the input variable to a blood pressure value as the output variable.

9. Blood pressure measurement device according to claim 8, further comprising an interface (26), via which blood pressure values measured in the patient are transmitted to the controller (20), wherein the controller (20) is configured to provide or adjust the mathematical function on the basis of the measured blood pressure values and an associated pulse wave.

10. Blood pressure measurement device according to claim 8 or 9, further comprising a warning signal unit (24), which is configured to output a warning signal when the blood pressure value determined by the control unit (22) reaches a predetermined limit value, wherein in particular the predetermined limit value is determined with reference to a blood pressure value of a preceding cardiac cycle, in particular at the start of a medical treatment.

11. Dialysis system (10), comprising a blood pressure measurement device (14) according to any one of claims 8 to 10.

**Revendications**

1. Procédé de détermination d'une valeur de pression sanguine d'un patient, comprenant les étapes consistant à :

   - détecter une mesure non invasive d'un pouls du patient et déterminer un tracé de pouls du patient au moyen d'une unité de mesure (18) ;
   - déterminer, au moyen d'une unité de commande (20), un point de référence (RP) dans le tracé de pouls dans une zone de transition (ÜB) de la systole à la diastole d'un cycle cardiaque du patient, dans lequel le point de référence (RP) est un point de retournement présentant un taux de variation ou une inclinaison positive entre un minimum local et un maximum local ; et
   - déterminer, au moyen de l'unité de commande (20), la valeur de pression sanguine sur la base du point de référence déterminé (RP),

   **caractérisé en ce que** la détermination s'effectue à l'aide d'une fonction mathématique fournie, laquelle corrèle un quotient formé à partir de l'instant (t) et de l'amplitude (h) du point de référence (RP) en tant que grandeur d'entrée avec une valeur de pression sanguine en tant que grandeur de sortie.

2. Procédé selon la revendication 1, dans lequel la valeur de pression sanguine correspond à une valeur de pression sanguine systolique et/ou diastolique et/ou à une valeur de pression sanguine artérielle moyenne.

3. Procédé selon la revendication 1 ou 2, dans lequel le tracé de pouls est détecté sur la base de valeurs d'irrigation sanguine dépendant du temps et du volume au niveau d'une section de tissu du patient, en particulier par photopléthysmographie.

4. Procédé selon l'une quelconque des revendications 1 à 3, dans lequel le tracé de pouls est normalisé par amplitude et/ou temps, en particulier normalisé par durée de cycle cardiaque.

5. Procédé selon l'une quelconque des revendications précédentes, dans lequel la fonction mathématique est une fonction linéaire ou une fonction polynomiale.

6. Procédé selon l'une quelconque des revendications précédentes, dans lequel l'étape de fourniture de la fonction mathématique comprend les étapes partielles suivantes :

   - fournir au moins deux enregistrements comprenant une valeur de pression sanguine mesurée et un instant (t) associé et/ou une amplitude (h) associée d'un point de référence (RP) ; et
   - déterminer la fonction mathématique sur la base des enregistrements fournis, en particulier au moyen d'un procédé d'interpolation ou de régression.

7. Procédé selon l'une quelconque des revendications précédentes, comprenant en outre une étape d'émission d'un signal d'avertissement lorsque la valeur de pression sanguine déterminée atteint une valeur limite prédéterminée, dans lequel en particulier la valeur limite prédéterminée est déterminée par rapport à une valeur de pression sanguine d'un cycle cardiaque précédent, en particulier au début d'un traitement médical.

8. Appareil de mesure de pression sanguine (14) pour déterminer une valeur de pression sanguine d'un patient, comprenant :

   - une unité de mesure (18) pour déterminer un tracé de pouls du patient ; et
   - une unité de commande (20) pour déterminer un point de référence (RP) dans le tracé de pouls dans une zone de transition de la systole à la diastole d'un cycle cardiaque du patient, et pour déterminer la valeur de pression sanguine sur la base du point de référence (RP) déterminé, dans lequel l'unité de commande (20) est configurée pour déterminer un point de retournement présentant un taux de variation ou une inclinaison positive entre un minimum local et un maximum local en tant que point de référence (RP),

   **caractérisé en ce que** l'unité de commande est configurée pour déterminer en outre la valeur de pression sanguine à l'aide d'une fonction mathématique fournie qui corrèle un quotient formé à partir de l'instant (t) et de l'amplitude (h) du point de référence (RP) en tant que grandeur d'entrée avec une valeur de pression sanguine en tant que grandeur de sortie.

9. Appareil de mesure de pression sanguine selon la revendication 8, comprenant en outre une interface (26) par l'intermédiaire de laquelle des valeurs de pression sanguine mesurées sur le patient sont transmises à l'unité de commande (20), dans lequel l'unité de commande (20) est configurée pour fournir ou adapter la fonction mathématique sur la base des valeurs de pression sanguine mesurées et d'un tracé de pouls associé.

10. Appareil de mesure de pression sanguine selon la revendication 8 ou 9, comprenant en outre une unité de signal d'avertissement (24) qui est configurée pour émettre un signal d'avertissement lorsque la valeur de pression sanguine déterminée par l'unité de commande (22) atteint une valeur limite prédéterminée, dans lequel en particulier la valeur limite prédéterminée est fixée par rapport à une valeur de pression sanguine d'un cycle cardiaque précédent, en particulier au début d'un traitement médical.

11. Système de dialyse (10) comprenant un appareil de mesure de pression sanguine (14) selon l'une quelconque des revendications 8 à 10.

Fig. 1

Fig. 2

| S1 | Ermitteln eines den Pulsverlauf des Patienten anzeigenden Signals |
|----|---|

| S2 | Verarbeitung des Signals mittels Tiefpassfilterung zum Ermitteln eines Pulsverlaufs des Patienten |
|----|---|

| S3 | Berechnung einer ersten und zweiten Ableitung des Pulsverlaufs |
|----|---|

| S4 | Extrahieren einzelner, unterschiedlichen Herzzyklen zugehöriger Pulskurven |
|----|---|

| S5 | Offset-Eliminierung |
|----|---|

| S6 | Normierung der Pulskurven |
|----|---|

| S7 | Bestimmen eines Referenzpunktes und den dazugehörigen Referenzparametern |
|----|---|

| S8 | Berechnen eines Mittlerwertes für die jeweiligen Referenzparameter über mehrere aufeinanderfolgende Herzzyklen |
|----|---|

| S9 | Berechnen des Blutdruckwerts auf der Grundlage der bestimmten Referenzparameter |
|----|---|

Fig. 3

Fig. 4

Fig. 5

EP 3 917 386 B1

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3427651 A1
- GB 2552455 A
- WO 2015092753 A1

**In der Beschreibung aufgeführte Nicht-Patentliteratur**

- Cuffless Blood Pressure Estimation Algorithms for Continuous Health-Care Monitoring. **KACHUEE MOHAMMAD et al.** IEEE TRANSACTIONS ON BIOMEDICAL ENGINEERING. IEEE SERVICE CENTER, 01 April 2017, vol. 64, 859-869